# EUROPÄISCHE PATENTSCHRIFT

(11) **EP 1 049 674 B1**
(45) Veröffentlichungstag und Bekanntmachung des Hinweises auf die Patenterteilung: **28.02.2007**
(21) Anmeldenummer: 98966836.3
(22) Anmeldetag: 18.12.1998
(51) Int. Cl.: C07D 211/42, C07D 211/74, C07D 211/72, C07D 405/04

(54) **VERFAHREN ZUR HERSTELLUNG VON (-)CIS-3- HYDROXY-1- METHYL-4-(2, 4,6-TRIMETHOXYPHENYL)- PIPERIDIN**
METHOD FOR PRODUCING (-)CIS-3-HYDROXY-1-METHYL-4-(2,4,6-TRIMETHOXYPHENYL)-PIPERIDINE
PROCEDE DE PRODUCTION DE (-)CIS-3-HYDROXY-1-METHYL-4-(2,4,6-TRIMETOXYPHENYL)-PIPERIDINE

(30) Priorität: 23.01.1998 DE 19802449
(43) Veröffentlichungstag der Anmeldung: 08.11.2000
(73) Patentinhaber: Sanofi-Aventis Deutschland GmbH, 65929 Frankfurt am Main (DE)
(72) Erfinder: BREIPOHL, Gerhard, D-60529 Frankfurt am Main (DE); MICHALOWSKY, Jürgen, D-65779 Kelkheim (DE)
(86) Internationale Anmeldenummer: PCT/EP1998/008327
(87) Internationale Veröffentlichungsnummer: WO 1999/037615

(56) Entgegenhaltungen:
- EP-A- 0 241 003
- EP-A- 0 366 061
- WO-A-98/13344
- US-A- 5 849 733
- SEDLACEK ET AL: "Flavopiridol (L86 8275; NSC 649890), a new kinase inhibitor for tumor therapy" INTERNATIONAL JOURNAL OF ONCOLOGY, Bd. 9, 1996, Seiten 1143-1168, XP002103774 in der Anmeldung erwähnt
- NAIK ET AL: "An antiinflammatory and immunomodulatory piperidinylbenzopyranone from Dysoxylum binectariferum: isolation, structure and total synthesis" TETRAHEDRON, Bd. 44, Nr. 7, 1. Januar 1988, Seiten 2081-2086, XP002093909

## Beschreibung

(-)cis-3-Hydroxy-1-methyl-4-(2,4,6-trimethoxyphenyl)-piperidin (II) ist ein zentraler Baustein in der Synthese des Flavopiridol(1) (HMR 1275 oder L86-8275), des ersten potenten Inhibitors der cyclin-abhängigen Protein-Kinase (siehe z.B. Sedlacek, Hans Harald; Czech, Joerg; Naik, Ramachandra; Kaur, Gurmeet; Worland, Peter; Losiewicz, Michael; Parker, Bernard; Carlson, Bradley; Smith, Adaline; et al. Flavopiridol (L86 8275; NSC 649890), a new kinase inhibitor for tumor therapy. Int. J. Oncol. (1996), 9(6), 1143-1168 oder Czech, Joerg; Hoffmann, Dieter; Naik, Ramachandra; Sedlacek, Hans-Harald. Antitumoral activity of flavone L 86-8275. Int. J. Oncol. (1995), 6(1), 31-36).

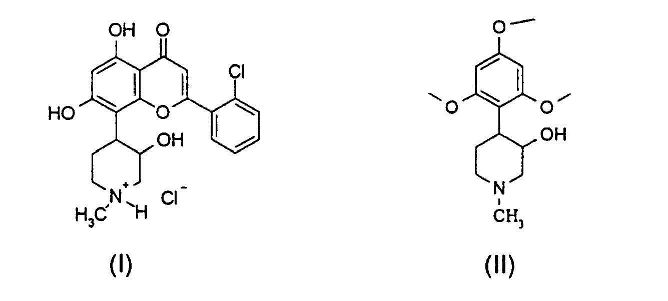

Das bisherige, in EP-B 0 241 003 und EP-B 0 366 061 beschriebene Verfahren zur Herstellung von (II) ist zeitaufwendig und beinhaltet Reaktionen (Hydroborierung, Swern-Oxidation, Natriumborhydrid-Reduktion) die im technischen Maßstab schwierig zu handhaben sind. Überraschenderweise haben wir nun ein wesentlich einfacheres Herstell-Verfahren gefunden, das in Schema 1 verdeutlicht ist.

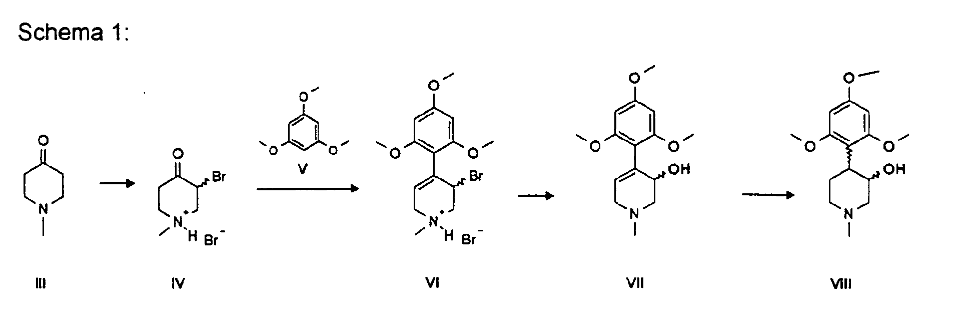

Das Verfahren zur Herstellung von (-)cis-3-Hydroxy-1-methyl-4-(2,4,6-trimethoxyphenyl)-piperidin(11) 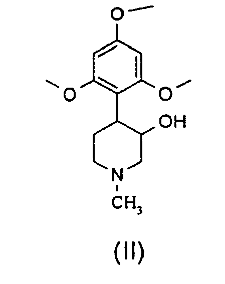 ist dadurch gekennzeichnet, daß man
a₁) 1-Methyl-piperidin-4-on (III) in das Hydrobromid nach bekannten Verfahren überführt, oder 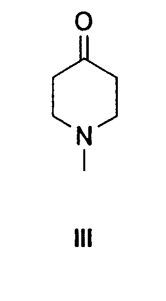
a₂) 1-Methyl-piperidin-4-on (III) direkt vor der nachfolgenden Bromierung durch Einbringen von 1-Methyl-piperidin-4-on in eine HBr-Eisessig-Lösung in das Hydrobromid überführt, und
b) 1-Methyl-piperidin-4-on-hydrobromid in einem geeigneten Lösungsmittel, wie z.B. Essigsäure, im Temperaturbereich von 0°-30°C mit Brom zum 3(R,S)-Brom-1-methyl-4-oxo-piperidin-hydrobromid (IV) umsetzt, 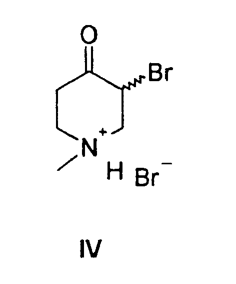
c) dieses Zwischenprodukt (IV) direkt durch Zugabe von 0.8-1 Äquivalenten 1,3,5-Trimethoxybenzol (V) 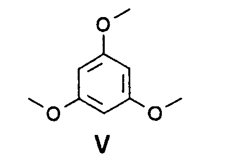 zur Reaktionslösung bei 0°-30°C zum 3(R,S)-Brom-1-methyl-4-(2,4,6-trimethoxyphenyl)-1,2,3,6-tetrahydro-pyridin-hydrobromid (VI) umsetzt, und gegebenenfalls zur Entfernung entstehenden Reaktionswassers noch Acetanhydrid zugibt, 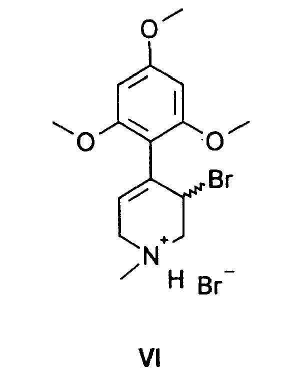
d₁) die Verbindung (V1) durch Einrühren der Reaktionslösung in ein geeignetes organisches Lösungsmittel, wie z.B. Methyl-tert.butylether, Dichlormethan etc., zunächst als Feststoff isoliert und nachfolgend das erhaltene Produkt mit Wasser versetzt und durch Rühren bei 50°-100°C, bevorzugt bei 60°-80°C, zu 3(R,S)-Hydroxy-1-methyl-4-(2,4,6-trimethoxyphenyl)-1,2,3,6-tetrahydro-pyridin (VII) umsetzt, oder 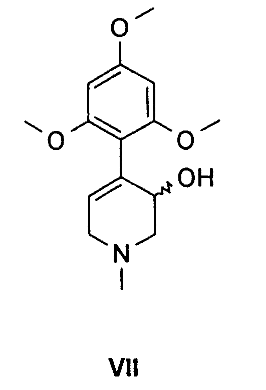
d₂) das die Verbindung (VI) enthaltende Reaktionsgemisch direkt mit Wasser versetzt und durch Rühren bei 50°-100°C, bevorzugt bei 60°-80°C, zu 3(R,S)-Hydroxy-1-methyl-4-(2,4,6-trimethoxyphenyl)-1 ,2,3,6-tetrahydro-pyridin (VII) umsetzt, und
d₃) die nach d₁) bzw. d₂) erhaltenen Reaktionsgemische abkühlt, gegebenenfalls weiter mit Wasser verdünnt und bei 0°-30°C durch Zugabe von wäßrigem Alkali, vorzugsweise Natriumhydroxid, auf pH >12 stellt, wobei 3(R,S)-Hydroxy-l-methyl-4-(2,4,6-trimethoxyphenyl)-1,2,3,6-tetrahydro-pyridin (VII) ausfällt, das erhaltene Rohprodukt absaugt und gegebenenfalls zur Reinigung erneut in wäßriger Salzsäure aufnimmt, filtriert und gegebenenfalls mit einem mit Wasser nicht mischbaren Lösungsmittel, z.B. Ethylacetat, extrahiert und anschließend die wäßrige Phase durch Zugabe von wäßrigem Alkali, vorzugsweise Natriumhydroxid, auf pH >12 stellt, wobei 3(R,S)-Hydroxy-1-methyl-4-(2,4,6-trimethoxyphenyl)-1,2,3,6-tetrahydro-pyridin (VII) ausfällt, das ausgefallene Produkt gegebenenfalls zur weiteren Reinigung mit einem oder mehreren geeigneten organischen Lösungsmitteln, wie z.B. Aceton, Isopropanol, Diisopropylether oder auch Gemischen dieser Lösungsmittel, ausrührt, und
e₁) das erhaltene Produkt (VII) in Methanol, Isopropanol, Wasser oder Gemischen aus diesen Lösungsmitteln mit Palladium/Kohle oder Rhodium/Kvhle als Katalysator zum racemischen 3,4-cis-Alkohol (VIII) katalytisch hydriert, 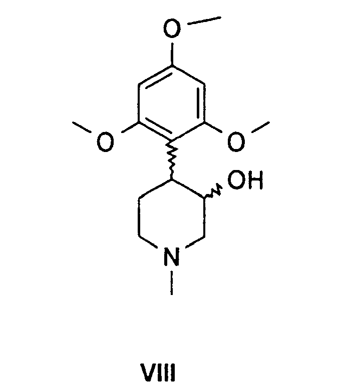 wobei der bei der Reduktion eventuell in geringen Mengen entstehende 3,4-trans Alkohol durch Kristallisation aus geeigneten Lösungsmitteln, wie z.B. Aceton, entfernt werden kann, oder
e₂) für die Hydrierung aus der Verbindung (VII) leicht zugängliche Ester (IXa) oder Carbonate (IXb) einsetzt, 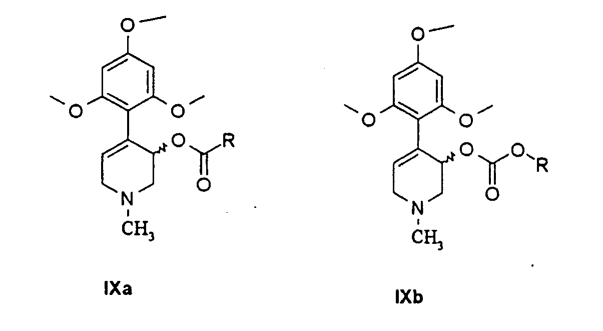 worin R (C₁-C₁₆)-Alkyl, (C₆-C₁₄)-Aryl-(C₁-C₁₆)-alkyl oder (C₆-C₁₄)-Aryl und in der Formel (IXa) ferner Carboxy-(C₂-C₆)-Alkyl bedeutet,
   wobei man Verbindungen (Xa) bzw. (Xb) erhält, aus denen nach bekannten Verfahren die Verbindung (VIII) freigesetzt werden kann,
f₁) durch Racematspaltung mit geeigneten chiralen Hilfsreagenzien wie z.B. Ketopinsäure nach bekanntem Verfahren aus der Verbindung (VIII) den enantiomerenreinen cis-Alkohol (11) erhält, oder
f₂) die Racematspaltung mit den Verbindungen (Xa) bzw. (Xb) durchführt 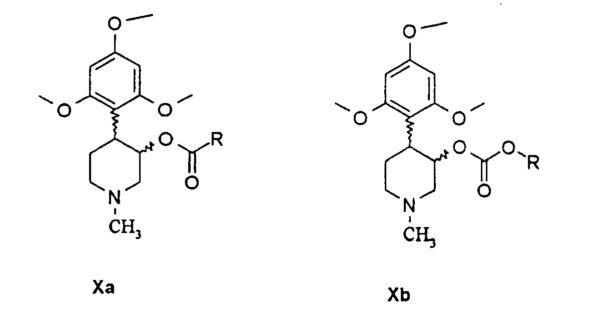 wobei R (C₁-C₁₆)-Alkyl, (C₆-C₁₄)-Aryl-(Ci-C₁₆)-alkyl oder (C₆-C₁₄)-Aryl und in der Formel (Xa) ferner Carboxy-(C₂-C₆)-Alkyl bedeutet,
   und diese dann nach bekannten Verfahren in die Verbindung (II) umwandelt,
   wobei die Reihenfolge der Reaktionsschritte e) und f) auch vertauscht sein kann, d.h. die Racematspaltung auch schon auf der Stufe des Allylalkohols (VII) oder der daraus erhaltenen Verbindungen (IXa) bzw. (IXb) durchgeführt werden kann.

Die Ester (IXa) oder Carbonate (IXb) eignen sich in Analogie zu einem in der Literatur beschriebenen Verfahren (Trost et al., JACS 1994, 116, 10320) zur Deracemisierung zu den enantiomerenreinen Estern. Nach Hydrierung und Esterspaltung erhält man dann (-)cis-3-Hydroxy-1-methyl-4-(2,4,6-trimethoxyphenyl)-piperidin (II).

3(R,S)-Brom-1-methyl-4-(2,4,6-trimethoxyphenyl)-1,2,3,6-tetrahydro-pyridin-hydrobromid (VI) und 3(R,S)-Hydroxy-1-methyl-4-(2,4,6-trimethoxyphenyl)-1,2,3,6-tetrahydro-pyridin (Vll) sind wertvolle Zwischenprodukte bei der Herstellung von (-)cis-3-Hydroxy-1-methyl-4-(2,4,6-trimethoxyphenyl)-piperidin (II).

### Beispiele:

### Beispiel 1:

### Herstellung von 3(R,S)-Brom-1-methyl-4-(2,4,6-trimethoxyphenyl)-1,2,3,6-tetrahydro-pyridin-hydrobromid(VI)

Zu 200 ml Eisessig wurden 75.7 ml (0.44 Mol) 33%ige HBr in Eisessig gegeben und dann unter Eiskühlung bei 20-25°C zügig 50 g (0.44 Mol) 1-Methyl-piperidin-4-on (III) zugetropft. In die hierbei erhaltene Suspension des Hydrobromides tropfte man während 30 min bei 20-25°C 70.4g (0.44 Mol) Brom zu wobei man eine klare, gelbliche Lösung erhielt. Man rührte noch 15 min bei 25°C nach und gab dann 67.2 g (0.40 Mol) 1,3,5-Trimethoxybenzol(V) zur Reaktionslösung. Dann wurde 1 h bei 25°C nachgerührt. Danach ließ man 300 ml Methyl-tert.butylether zulaufen, wobei das Produkt ölig ausfiel. Man dekantierte den Überstand ab, verrührte erneut mit 300 ml Methyl-tert.butylether und dekantierte ab. Der Rückstand wurde dann mit 150 ml Dichlormethan verrührt, wobei das Produkt auskristallisierte. Das erhaltene 3(R,S)-Brom-1-methyl-4-(2,4,6-trimethoxyphenyl)-1,2,3,6-tetrahydro-pyridin-hydrobromid(VI) wurde abgesaugt, mit 50 ml Dichlormethan gewaschen und im Vakuum getrocknet.
Ausbeute: 147 g fast farblose Kristalle.
Schmp.: 190-192°C
MS(ES⁺): 342.2(M+H)⁺

### Beispiel 2:

### Herstellung von 3(R,S)-Hydroxy-1-methyl-4-(2,4,6-trimethoxyphenyl)-1,2,3,6-tetrahydro-pyridin(VII)

50 g (0.118 Mol) 3(R,S)-Brom-1-methyl-4-(2,4,6-trimethoxyphenyl)-1,2,3,6-tetrahydro-pyridin-hydrobromid (V1) wurden in 100 ml Wasser 2h unter Rückfluß erhitzt. Dann wurde das Reaktionsgemisch auf 20°C abgekühlt und durch Zutropfen von 30 ml 30%iger Natronlauge auf pH 12,5 gestellt. Nach kurzer Zeit kristallisierte ein hellbrauner Niederschlag aus. Man rührte noch 1 h bei 5-10°C nach, saugte das ausgefallene Produkt ab und wusch mit 30 ml Wasser nach. Das Rohprodukt wurde dann mit 20 ml Aceton verrührt, abgesaugt und im Vakuum getrocknet.
Ausbeute: 25.2 g farblose Kristalle
Schmp.: 125-127°C
MS(Cl⁺): 280.3(M+H)⁺

### Beispiel 3:

### Herstellung von 3(R,S)-Hydroxy-1-methyl-4-(2,4,6-trimethoxyphenyl)-1 ,2,3,6-tetrahydro-Pyridin (VII)

Zu 100 ml Eisessig wurden 75.7 ml (0.44 Mol) 33%ige HBr in Eisessig gegeben und dann unter Eiskühlung bei 20-25°C zügig 50 g (0.44 Mol) 1-Methyl-piperidin-4-on (III) zugetropft. In die hierbei erhaltene Suspension des Hydrobromides tropfte man unter Stickstoff während 30 min bei 20-25°C 70.4g (0.44 Mol) Brom zu, wobei man eine klare, gelbliche Lösung erhielt. Man rührte noch 60 min bei 25°C nach und gab dann 67.2 g (0.40 Mol) 1,3,5-Trimethoxybenzol (V) zur Reaktionslösung. Dann wurde 1 h bei 25°C nachgerührt. Danach ließ man 400 ml Wasser zulaufen und erhitzte das Gemisch 3h zum Rückfluß. Man ließ das Reaktiongemisch über Nacht bei Raumtemperatur stehen, verdünnte mit 400 ml Wasser, kühlte dann auf 10°C und tropfte während 4h bei dieser Temperatur insgesamt 320 ml konz. Natronlauge zur gut gerührten Mischung (pH nach Zugabe 10.7). Dabei fiel der Allylalkohol (VII) zunächst leicht schmierig aus, wurde aber bei längerem Rühren dann fest und gut absaugbar. Der dunkelgelbe Niederschlag wurde abgesaugt, mit Wasser gewaschen und gut getrocknet. Das Rohprodukt wurde anschließend mit 80 ml Aceton verrührt, abgesaugt und im Vakuum getrocknet.
Ausbeute: 55 g hellgelbe Kristalle
Schmp.: 125-127°C
MS(Cl⁺): 280.3(M+H)⁺
¹H-NMR(dmso-d₆): δ (ppm)6.15(s, 2H); 5.65(dd, 1 H); 4.20(m, 1 H); 3.80(s, 3H); 3.75(s, 6H); 3.31 (dd1 H); 2.89(m, 1 H); 2.84(dd, 1 H); 2.71 (d, 1 H); 2.57(dd, 1 H); 2.24(s, 3H)

### Beispiel 4:

### Herstellung von 3(R,S)-Hydroxy-1-methyl-4-(2,4,6-trimethoxyphenyl)-1,2,3,6-tetrahydro-pyridin (VII)

Zu 100 ml Eisessig wurden 75.7 ml (0.44 Mol) 33%ige HBr in Eisessig gegeben und dann unter Eiskühlung bei 20-25°C zügig 50 g (0.44 Mol) 1-Methyl-piperidin-4-on (III) zugetropft. In die hierbei erhaltene Suspension des Hydrobromides tropfte man unter Stickstoff während 30 min bei 20-25°C 70.4g (0.44 Mol) Brom zu, wobei man eine klare, gelbliche Lösung erhielt. Man rührte noch 60 min bei 25°C nach und gab dann 67.2 g (0.40 Mol) 1,3,5-Trimethoxybenzol (V) zur Reaktionslösung. Nach 15 min wurden unter Kühlung noch 40.8 g (0.4 Mol) Acetanhydrid zugegeben und 1 h bei 25°C nachgerührt. Danach ließ man 750 ml Wasser zulaufen und erhitzte das Gemisch 9.5h auf 80°C. Das Reaktiongemisch wurde dann auf 5-10°C gekühlt und durch Zutropfen von 200 ml konz. Natronlauge innerhalb von 30 min auf pH 5.5 gestellt. Dabei fiel nicht umgesetztes 1,3,5-Trimethoxybenzol aus. Die Suspension wurde filtriert und das Filtrat mit weiteren 200 ml konz. Natronlauge bei 5-10°C auf pH 14 gestellt. Dabei fiel der Allylalkohol (VII) zunächst leicht schmierig aus, wurde aber bei längerem Rühren fest und dann gut absaugbar. Der Ansatz stand über Nacht bei Raumtemperatur. Dann wurde der hellgelbe Niederschlag abgesaugt, mit ca. 300 ml Wasser bis zum neutralen pH gewaschen und gut getrocknet. Rohausbeute: 87.2 g hellgelbe Kristalle.
Das Rohprodukt wurde anschließend mit 100 ml Aceton verrührt, abgesaugt und im Vakuum getrocknet.
Ausbeute: 73 g schwachgelbe Kristalle
Schmp.: 125-127°C
MS(Cl⁺): 280.3(M+H) ⁺

### Beispiel 5:

### Herstellung von racemischem 3,4-cis-Alkohol (VIII)

3.5 g (12.5 mMol) 3(R,S)-Hydroxy-1-methyl-4-(2,4,6-trimethoxyphenyl)-1,2,3,6-tetrahydro-pyridin (VII) wurden in 100 ml Methanol gelöst und mit 0.35 g Katalysator (5% Pd/C, zuvor mit MeOH gewaschen) versetzt und im Büchi-Autoklaven 15h bei 50°C und 50 bar Wasserstoffdruck hydriert. Dann wurde der Katalysator abfiltriert, das Filtrat im Vakuum am Rotationsverdampfer eingedampft und der Rückstand mit 4 ml Aceton verrührt. Das erhaltene Produkt wurde abfiltriert und im Vakuum getrocknet.
Ausbeute: 2.7 g farblose Kristalle
Schmp.: 131-132°C
MS(Cl⁺): 282.3(M+H)⁺

### Beispiel 6:

### Herstellung von racemischem 3,4-cis-Alkohol (VIII)

10.0 g (35.8 mMol) 3(R,S)-Hydroxy-1-methyl-4-(2,4,6-trimethoxyphenyl)-1,2,3,6-tetrahydro-pyridin (VII) wurden in 100 ml Methanol gelöst und mit 1.5 g Katalysator (5% Pd/C, zuvor mit MeOH gewaschen) versetzt und im Büchi-Autoklaven 39h bei 50°C und 50 bar Wasserstoffdruck hydriert. Danach wurde der Katalysator abfiltriert, das Filtrat im Vakuum am Rotationsverdampfer eingedampft und der Rückstand mit 20 ml Aceton verrührt. Das erhaltene Produkt wurde abfiltriert und im Vakuum getrocknet.
Ausbeute: 9.2 g farblose Kristalle
Schmp.: 131-132°C
MS(Cl⁺): 282.3(M+H)⁺

### Beispiel 7:

### Herstellung von 3(R,S)-Acetoxy-1-methyl-4-(2,4,6-trimethoxyphenyl)-1,2,3,6-tetrahydro-pyridin (lXa) [R= Methyl]

2.79 g (10 mMol) 3(R,S)-Hydroxy-1-methyl-4-(2,4,6-trimethoxyphenyl)-1,2,3,6-tetrahydro-pyridin (Vll) wurden in 15 ml Acetanhydrid gelöst und 4h bei 100°C gerührt. Die Lösung wurde dann im Vakuum am Rotationsverdampfer eingedampft und der Rückstand in 10 ml Wasser gelöst, mit Natronlauge auf pH>12 gestellt und 2mal mit je 20 ml Ethylacetat extrahiert. Die vereinigten organischen Phasen wurden mit 10 ml gesättigter Natriumchlorid-Lösung gewaschen, dann über Natriumsulfat getrocknet und im Vakuum am Rotationsverdampfer eingedampft.
Ausbeute: 2.55 g eines Öles
MS(ES⁺): 322.2(M+H)⁺

### Beispiel 8:

### Herstellung von 3(R,S)-Methyloxycarbonyloxy-1-methyl-4-(2,4,6-trimethoxyphenyl)-1,2,3,6-tetrahydro-pyridin (IXb) [R= Methyl]

8.37 g (30 mMol) 3(R,S)-Hydroxy-1-methyl-4-(2,4,6-trimethoxyphenyl)-1,2,3,6-tetrahydro-pyridin (VII) wurden in 83.7 ml Tetrahydrofuran gelöst und mit 12.4 ml (90 mMol) Triethylamin versetzt. Man kühlte auf 5°C ab, tropfte während 15 min 2.55 ml (33 mMol) Chlorameisensäuremethylester zu und rührte noch 3h bei 5-10°C nach. Da die DC-Kontrolle noch Edukt anzeigte, wurden weitere 1.0 ml (12.9 mMol) Chlorameisensäuremethylester zugefügt und nochmals 1 h nachgerührt. Dann wurden 50 ml Wasser zugefügt und 2mal mit je 50 ml Ethylacetat extrahiert. Die vereinigten organischen Phasen wurden mit 30 ml gesättigter Natriumchlorid-Lösung gewaschen, dann über Natriumsulfat getrocknet und im Vakuum am Rotationsverdampfer eingedampft.
Ausbeute: 8.7 g eines Öles das beim Stehenlassen fest wird
MS(ES⁺): 338.2(M+H)⁺

### Beispiel 9:

### Herstellung von racemischem cis-Ester (Xa) [R= Methyl]

15.33 g Toluolsulfonsäure-Salz von 3(R,S)-Acetoxy-1-methyl-4-(2,4,6-trimethoxyphenyl)-1,2,3,6-tetrahydro-pyridin (IXa) [R= Methyl] wurden in 153 ml Methanol gelöst, über Aktivkohle filtriert und das Filtrat mit 1.53 g Katalysator- (5% Rh/C) versetzt und im Büchi-Autoklaven 24h bei 50°C und 18 bar Wasserstoffdruck hydriert. Danach wurde der Katalysator abfiltriert und das Filtrat im Vakuum am Rotationsverdampfer eingedampft. Der Rückstand wurde in 50 ml Wasser gelöst und mit konz. Natronlauge ein pH-Wert von >12 eingestellt. Nach kurzer Zeit fiel die Base aus. Das erhaltene Produkt wurde abfiltriert, mit Wasser gewaschen und im Vakuum getrocknet.
Ausbeute: 8.5 g farblose Kristalle
Schmp.: 115-117°C
MS(Cl⁺): 324.2(M+H)⁺

### Beispiel 10:

### Herstellung von racemischem 3,4-cis-Alkohol (VIII) aus (Xa) [R= Methyl]

1.61 g (5 mmol) racemischer cis-Ester (Xa) [R= Methyl] wurden in 20 ml Methanol und mit 5 ml konz. Salzsäure 8h unter Rückfluß erhitzt. Dann wurde das Methanol im Vakuum entfernt, der Rückstand mit 10 ml Wasser versetzt und mit konz. Natronlauge ein pH-Wert von >12 eingestellt. Dabei fiel das Produkt aus. Das erhaltene Produkt wurde abfiltriert, mit Wasser gewaschen und im Vakuum getrocknet.
Ausbeute: 1.1 g farblose Kristalle
Schmp.: 131-132°C
MS(Cl⁺): 282.4(M+H)⁺

## Patentansprüche

1. Verfahren zur Herstellung von (-)cis-3-Hydroxy-1-methyl-4-(2,4,6-trimethoxyphenyl)-piperidin(II) 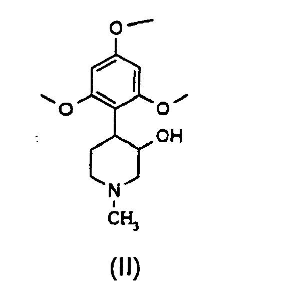 **dadurch gekennzeichnet, daß** man
a₁) 1-Methyl-piperidin-4-on (III) in das Hydrobromid nach bekannten Verfahren überführt, oder 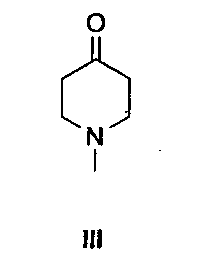
a₂) 1-Methyl-piperidin-4-on (III) direkt vor der nachfolgenden Bromierung durch Einbringen von 1-Methyl-piperidin-4-on in eine HBr-Eisessig-Lösung in das Hydrobromid überführt, und
b) 1-Methyl-piperidin-4-on-hydrobromid in einem geeigneten Lösungsmittel im Temperaturbereich von 0°-30°C mit Brom zum 3(R,S)-Brom-1-methyl-4-oxopiperidin-hydrobromid (IV) umsetzt, 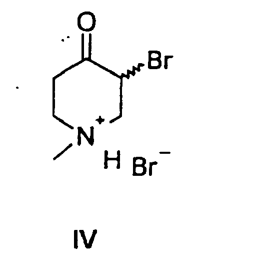
c) dieses Zwischenprodukt (IV) direkt durch Zugabe von 0.8-1 Äquivalenten 1,3,5-Trimethoxybenzol (V) 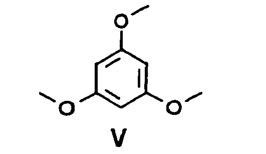 zur Reaktionslösung bei 0°-30°C zum 3(R,S)-Brom-1-methyl-4-(2,4,6-trimethoxyphenyl)-1,2,3,6-tetrahydro-pyridin-hydrobromid (VI) umsetzt, und gegebenenfalls zur Entfernung entstehenden Reaktionswassers noch Acetanhydrid zugibt, 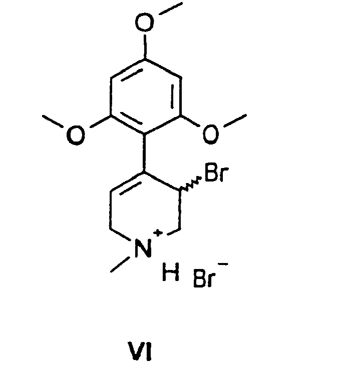
d₁) die Verbindung (VI) durch Einrühren der Reaktionslösung in ein geeignetes organisches Lösungsmittel zunächst als Feststoff isoliert und nachfolgend das erhaltene Produkt mit Wasser versetzt und durch Rühren bei 50°-100°C zu 3(R, S)-Hydroxy-1-methyl-4-(2,4,6-trimethoxyphenyl)-1,2, 3,6-tetrahydro-pyridin (VII) umsetzt, oder 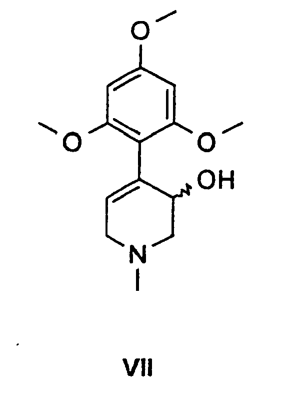
d₂) das die Verbindung (VI) enthaltende Reaktionsgemisch direkt mit Wasser versetzt und durch Rühren bei 50°-100°C zu 3(R,S)-Hydroxy-1-methyl-4-(2,4,6-trimethoxyphenyl)-1,2,3,6-tetrahydro-pyridin (VII) umsetzt, und
d₃) die nach d₁) bzw. d₂) erhaltenen Reaktionsgemische abkühlt, gegebenenfalls weiter mit Wasser verdünnt und bei 0°-30°C durch Zugabe von wäßrigem Alkali auf pH >12 stellt, wobei 3(R,S)-Hydroxy-1-methyl-4-(2,4,6-trimethoxyphenyl)-1,2,3,6-tetrahydro-pyridin (VII) ausfällt,
das erhaltene Rohprodukt absaugt und gegebenenfalls zur Reinigung erneut in wäßriger Salzsäure aufnimmt, filtriert und gegebenenfalls mit einem mit Wasser nicht mischbaren Lösungsmittel extrahiert und anschließend die wäßrige Phase durch Zugabe von wäßrigem Alkali auf pH >12 stellt, wobei 3(R,S)-Hydroxy-1-methyl-4-(2,4,6-trimethoxyphenyl)-1,2,3,6-tetrahydro-pyridin (Vll) ausfällt, das ausgefallene Produkt gegebenenfalls zur weiteren Reinigung mit einem oder mehreren geeigneten organischen Lösungsmitteln oder auch Gemischen dieser Lösungsmittel, ausrührt, und
e₁) das erhaltene Produkt (VII) in Methanol, Isopropanol, Wasser oder Gemischen aus diesen Lösungsmitteln mit Palladium/Kohle oder Rhodium/Kohle als Katalysator zum racemischen 3,4-cis-Alkohol (VIII) katalytisch hydriert, 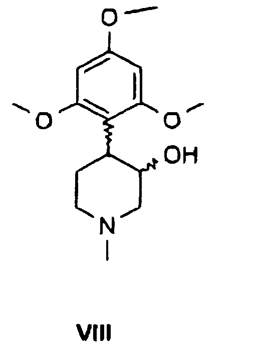 wobei der bei der Reduktion eventuell in geringen Mengen entstehende 3,4-trans Alkohol durch Kristallisation aus geeigneten Lösungsmitteln entfernt werden kann, oder
e₂) für die Hydrierung aus der Verbindung (VII) leicht zugängliche Ester (IXa) oder Carbonate (IXb) einsetzt, 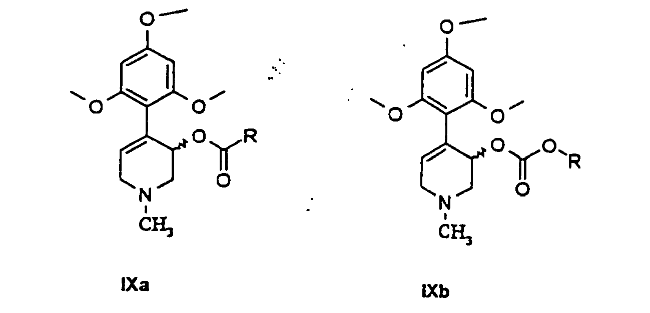 worin R (C₁-C₁₆)-Alkyl, (C₆-C₁₄)-Aryl-(C₁-C₁₆)-alkyl oder (C₆-C₁₄)-Aryl und in der Formel (IXa) ferner Carboxy-(C₂-C₆)-Alkyl bedeutet,
wobei man Verbindungen (Xa) bzw. (Xb) erhält, aus denen nach bekannten Verfahren die Verbindung (VIII) freigesetzt werden kann,
f₁) durch Racematspaltung mit geeigneten chiralen Hilfsreagenzien nach bekanntem Verfahren aus der Verbindung (VIII) den enantiomerenreinen cis-Alkohol (II) erhält, oder
f₂) die Racematspaltung mit den Verbindungen (Xa).bzw. (Xb) durchführt 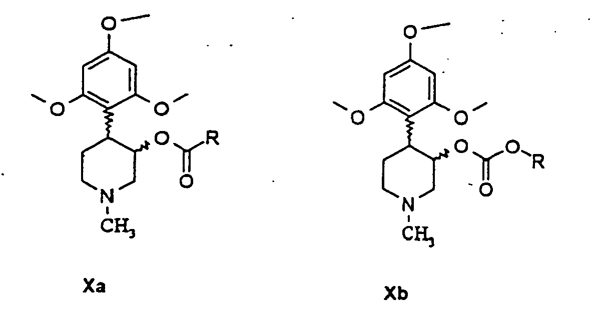 wobei R (C₁-C₁₆)-Alkyl, (C₆-C₁₄)-Aryl-(C₁-C₁₆)-alkyl oder (C₆-C₁₄)-Aryl und in der Formel (Xa) ferner Carboxy-(C₂-C₆)Alkyl bedeutet,
und diese dann nach bekannten Verfahren in die Verbindung (II) umwandelt,
wobei die Reihenfolge der Reaktionsschritte e) und f) auch vertauscht sein kann, d.h. die Racematspaltung auch schon auf der Stufe des Allylalkohols (VII) oder der daraus erhaltenen Verbindungen (IXa) bzw. (IXb) durchgeführt werden kann.

2. 3(R, S)-Brom-1-methyl-4-(2,4, 6-trimethoxyphenyl)-1,2, 3,6-tetrahydro-pyridinhydrobromid(VI) .

3. 3(R, S)-Hydroxy-1-methyl-4-(2,4,6-trimethoxyphanyl)-1,2,3,6-tetrehydro-pyridin(VII).

## Claims

1. A process for the preparation of (-)cis-3-hydroxy-1-methyl-4-(2,4,6-trimethoxyphenyl)piperidine (II) 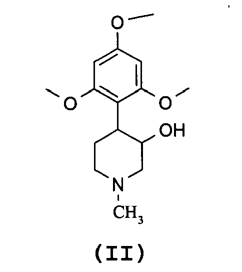 which comprises
a₁) converting 1-methylpiperidin-4-one (III) 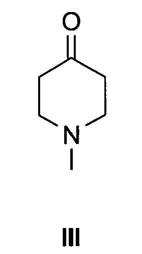 into its hydrobromide according to known processes, or
a₂) converting 1-methylpiperidin-4-one (III) directly into the hydrobromide, prior to the subsequent bromination, by adding 1-methylpiperidin-4-one into an HBr glacial acetic acid solution, and
b) reacting 1-methylpiperidin-4-one hydrobromide in a suitable solvent in the temperature range from 0°C-30°C with bromine to give 3-(R,S)-bromo-1-methyl-4-oxopiperidine hydrobromide (IV) 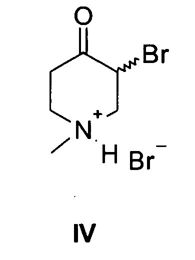
c) reacting this intermediate (IV) directly by addition of 0.8-1 equivalent of 1,3,5-trimethoxybenzene (V) 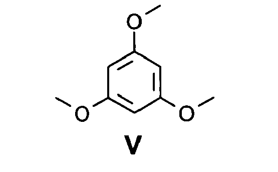 to the reaction solution at 0°-30°C to give 3-(R,S)-bromo-1-methyl-4-(2,4,6-trimethoxyphenyl)-1,2,3,6-tetrahydropyridine hydrobromide (VI) 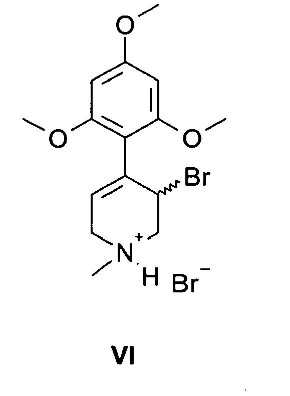 and, if appropriate, additionally adding acetic anhydride to remove any water forming during the reaction
d₁) first isolating the compound (VI) as a solid by stirring the reaction solution into a suitable organic solvent, and subsequently reacting the resulting product with water and by stirring at 50°-100°C to give 3-(R,S)-hydroxy-l-methyl-4-(2,4,6-trimethoxyphenyl)-1,2,3,6-tetrahydropyridine (VII) 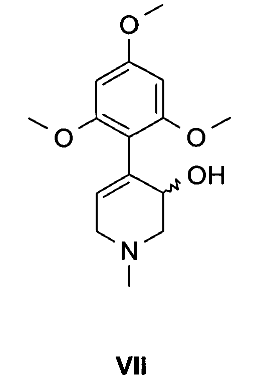 or
d₂) treating the reaction mixture containing the compound (VI) directly with water and reacting by stirring at 50°-100°C to give 3-(R,S)-hydroxy-1-methyl-4-(2,4,6-trimethoxyphenyl)-1,2,3,6-tetrahydropyridine (VII), and
d₃) cooling the reaction mixtures obtained according to d₁) or d₂), if appropriate diluting further with water and adjusting to pH > 12 at 0°-30°C by addition of aqueous alkali, 3-(R,S)-hydroxy-1-methyl-4-(2,4,6-trimethoxyphenyl)-1,2,3,6-tetrahydropyridine (VII) precipitating, filtering off the resulting crude product with suction and if appropriate, for purification, taking it up again in aqueous hydrochloric acid, filtering and optionally extracting with a water-immiscible solvent, and then adjusting the aqueous phase to pH > 12 by addition of aqueous alkali, 3-(R,S)-hydroxy-1-methyl-4-(2,4,6-trimethoxyphenyl)-1,2,3,6-tetrahydropyridine (VII) precipitating, extracting the precipitated product, if appropriate for further purification, by stirring with one or more suitable organic solvents or alternatively mixtures of these solvents, and
e₁) catalytically hydrogenating the resulting product (VII) in methanol, isopropanol, water or mixtures of these solvents using palladium/carbon or rhodium/carbon as catalyst to give the racemic 3,4-cis alcohol (VIII) 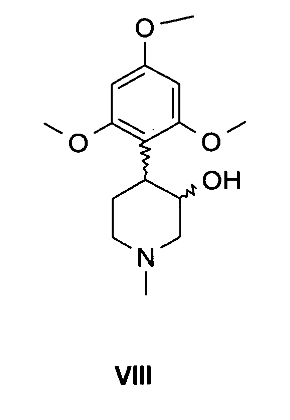 where the 3,4-trans alcohol, which possibly results in small amounts during the reduction, can be removed by crystallization from suitable solvents, or
e₂) for the hydrogenation, employing easily accessible esters (IXa) or carbonates (IXb) of the compound (VII) 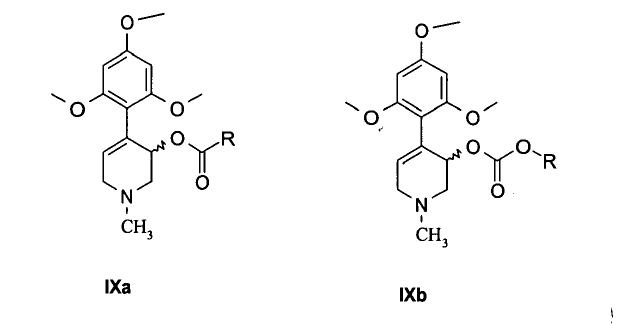 in which R is (C₁-C₁₆) -alkyl, (C₆-C₁₄) -aryl-(C₁-C₁₆)-alkyl or (C₆-C₁₄)-aryl and in the formula (IXa) is further carboxy- (C₂-C₆) -alkyl,
compounds (Xa) and (Xb) being obtained, from which the compound (VIII) can be cleaved by known procedures,
f₁) obtaining the enantiomerically pure cis alcohol (II) from the compound (VIII) by a known process by resolution using suitable chiral auxiliary reagents, or
f₂) carrying out the resolution using the compounds (Xa) or (Xb) 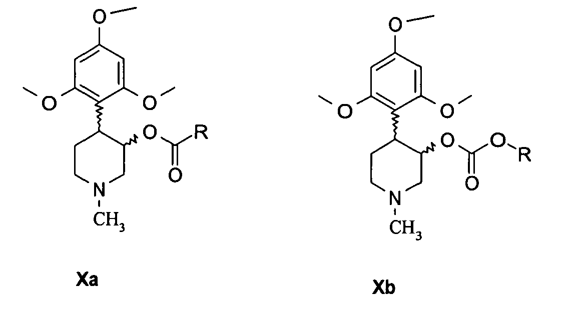 where R is (C₁-C₁₆) -alkyl, (C₆-C₁₄) -aryl- (C₁-C₁₆)-alkyl or (C₆-C₁₄) -aryl and in the formula (Xa) is further carboxy- (C₂-C₆) -alkyl,
and then converting these into the compound (II) by known processes,
it also being possible to exchange the sequence of the reaction steps e) and f), i.e. also to carry out the resolution as early as at the stage of the allyl alcohol (VII) or of the compounds (IXa) and (IXb) obtained therefrom.

2. 3-(R,S)-Bromo-l-methyl-4-(2,4,6-trimethoxyphenyl)-1,2,3,6-tetrahydropyridine hydrobromide (VI).

3. 3-(R,S)-Hydroxy-l-methyl-4-(2,4,6-trimethoxyphenyl)-1,2,3,6-tetrahydropyridine (VII).

## Revendications

1. Procédé pour la préparation de (-)cis-3-hydroxy-1-méthyl-4-(2,4,6-triméthoxyphényl)-pipéridine (II) 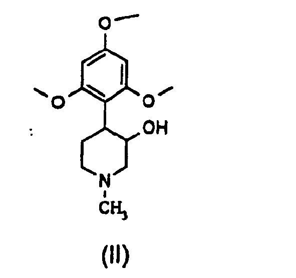 **caractérisé en ce que**
a1) on transforme de la 1-méthyl-pipéridin-4-one (III) en bromhydrate selon des procédés connus, ou 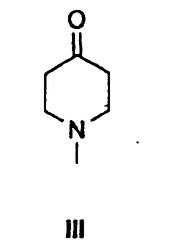
a2) on transforme la 1-méthyl-pipéridin-4-one (III) directement avant la bromation consécutive par introduction de 1-méthyl-pipéridin-4-one dans une solution de HBr-acide acétique glacial en bromhydrate, et
b) on transforme le bromhydrate de 1-méthyl-pipéridin-4-one dans un solvant approprié dans une plage de température de 0°-30°C avec du brome en bromhydrate de 3-(R,S)-bromo-1-méthyl-4-oxopipéridine (IV), 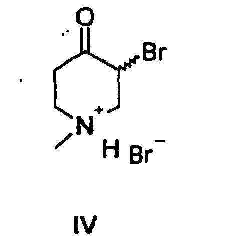
c) on transforme ce produit intermédiaire (IV) directement par addition de 0,8-1 équivalent de 1,3,5-triméthoxybenzène (V) 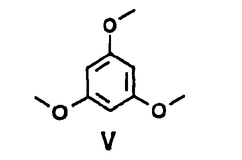 à la solution réactionnelle à 0°-30°C en bromhydrate de 3-(R,S)-bromo-1-méthyl-4-(2,4,6-triméthoxyphényl)-1,2,3, 6-tétrahydro-pyridine (VI) et on ajoute le cas échéant encore de l'anhydride de l'acide acétique pour l'élimination de l'eau réactionnelle formée, 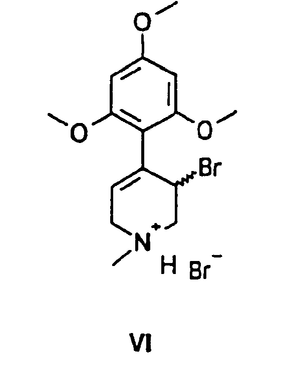
d1) on isole d'abord le composé (VI) en délayant la solution réactionnelle dans un solvant organique approprié sous forme solide, puis on mélange le produit obtenu avec de l'eau et on le transforme par agitation à 50°-100°C en 3-(R,S)-hydroxy-1-méthyl-4-(2,4,6-triméthoxyphényl)-1,2,3,6-tétrahydro-pyridine (VII), ou 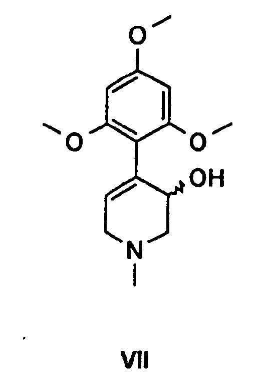
d2) on mélange le mélange réactionnel contenant le composé (VI) directement avec de l'eau et on le transforme par agitation à 50°-100°C en 3-(R,S)-hydroxy-1-méthyl-4-(2,4,6-triméthoxyphényl)-1,2,3,6-tétrahydro-pyridine (VII), et
d3) on refroidit les mélanges réactionnels obtenus selon les points d1) resp. d2), on les dilue le cas échéant davantage avec de l'eau et on les règle à un pH >12 à 0°-30°C par addition d'un alcalin aqueux, ce qui précipite la 3-(R,S)-hydroxy-1-méthyl-4-(2,4,6-triméthoxyphényl)-1,2,3,6-tétrahydro-pyridine (VII), on aspire le produit brut obtenu et on le reprend le cas échéant, pour la purification, à nouveau dans de l'acide chlorhydrique aqueux, on le filtre et on l'extrait le cas échéant avec un solvant non miscible avec l'eau, puis on règle la phase aqueuse par addition d'un alcalin aqueux à pH >12, ce qui précipite la 3-(R,S)-hydroxy-1-méthyl-4-(2,4,6-triméthoxyphényl)-1,2, 3,6-tétrahydro-pyridine (VII), on mélange le produit précipité le cas échéant, pour une nouvelle purification, avec un ou plusieurs solvants organiques appropriés ou également des mélanges de ces solvants, et
e1) on hydrogène catalytiquement le produit obtenu (VII) dans du méthanol, de l'isopropanol, de l'eau ou des mélanges de ces solvants avec du palladium/carbone ou du rhodium/carbone comme catalyseur en 3,4-cis-alcool racémique (VIII), 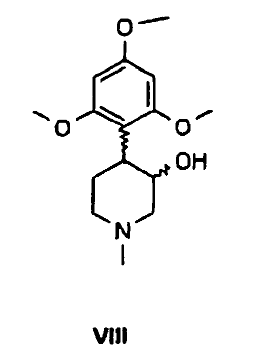 l'alcool 3,4-trans éventuellement formé en de faibles quantités lors de la réduction pouvant être enlevé par cristallisation à partir de solvants appropriés, ou
e2) on utilise pour l'hydrogénation des esters (IXa) ou des carbonates (IXb) facilement accessibles à partir du composé (VII), 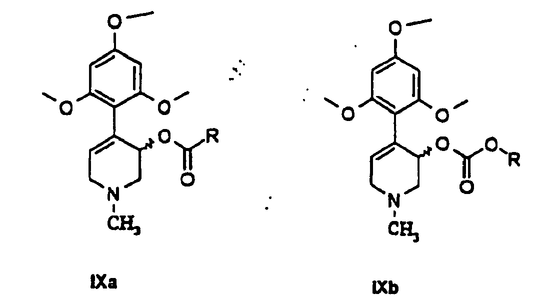 où R signifie (C1-C16)-alkyle, (C6-C14)-aryl-(C1-C16)-alkyle ou (C6-C14)-aryle et dans la formule (IXa) en outre carboxy-(C2-C6)-alkyle,
ce qui permet d'obtenir des composés (Xa) resp. (Xb) à partir desquels on peut libérer selon des procédés connus le composé (VIII),
f1) on obtient, par dissociation racémique avec des réactifs auxiliaires chiraux appropriés, selon des procédés connus, à partir du composé (VIII) le cis-alcool énantiomère pur (II), ou
f2) on réalise la dissociation du racémate avec les composés (Xa) resp. (Xb) 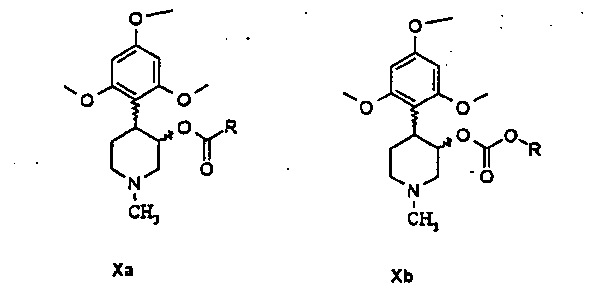 où R signifie (C1-C16) -alkyle, (C6-C14)-aryl-(C1-C16)-alkyle ou (C6-C14)-aryle et dans la formule (Xa) en outre carboxy-(C2-C6)-alkyle,
et on les transforme selon des procédés connus en composé (II),
l'ordre des étapes de réaction e) et f) pouvant également être échangé, c'est-à-dire que la dissociation du racémate peut également déjà être réalisée au niveau de l'étape de l'alcool allylique (VII) ou des composés (IXa) resp. (IXb) pouvant être obtenus à partir de celui-ci.

2. Bromhydrate de 3-(R,S)-bromo-1-méthyl-4-(2,4,6-triméthoxyphényl)-1,2,3,6-tétrahydro-pyridine (VI).

3. 3-(R,S)-hydroxy-1-méthyl-4-(2,4,6-triméthoxyphényl)-1,2,3,6-tétrahydropyridine (VII).
